# EUROPEAN PATENT APPLICATION

(11) **EP 2 070 559 A1**
(43) Date of publication of application: **17.06.2009**
(21) Application number: 08253952.9
(22) Date of filing: 10.12.2008
(51) Int. Cl.: A61M 5/32

(54) **Safety needle assembly**

(30) Priority: 14.12.2007 US 7657 P
(71) Applicant: Tyco Healthcare Group LP, Mansfield, MA 02048 (US)
(72) Inventor: Sansoucy, Michael, Wrentham, Massachusetts MA 02093 (US); Marquart, Carol A., Washington, MO 63090 (US)
(74) Representative: Pratt, David Martin

(57) **Abstract**

A safety needle assembly is disclosed which includes a housing, a needle extending distally from the housing, a slide member positioned within the housing and configured to move distally about the needle and at least one actuation member. The actuation member is supported on the housing and has a proximal end secured to the housing, a distal end obstructing distal movement of the slide member and a centrally located fulcrum. A biasing member is positioned within the housing to advance the slide member distally along the needle. A cover member is operably connected to the slide member and is configured to enclose a distal end of the needle. The actuation member is configured such that actuation of the actuation member pivots the distal end of the actuation member to facilitate distal movement of the slide member.

## Description

### CROSS-REFERENCE TO RELATED APPLICATION

The present application claims the benefit of and priority to U.S. Provisional Patent Application Serial No. 61/007,657 filed December 14, 2007, the entire contents of each are hereby incorporated by reference.

### BACKGROUND

### Technical Field

The present disclosure relates to safety needles for use in medical procedures, and more particularly, the present disclosure relates to a biased safety needle assembly.

### Background of Related Art

Hypodermic needles are used in wide range of fields, including, medicine, science, and veterinary medicine, and in industries such as, biotechnology, chemical and pharmaceutical. While safety needles would be welcomed by anyone in any of these professions, safety needles are particularly necessary in the healthcare profession. With the proliferation of HIV and serum hepatitis, needle stick injuries have life-threatening consequences and healthcare workers must live with this risk on a daily basis. Conventional safety needles have been developed to protect the healthcare worker from accidental needle stick injuries. However, the cost, ease of use and effectiveness of these safety needles leaves room for improvement.

Therefore, it would be beneficial to have a needle assembly that is easily activated by the user, adequately protects a user from accident needle stick injury and is economical to produce.

### SUMMARY

A safety needle assembly is disclosed which includes a housing, a needle extending distally from the housing, a slide member positioned within the housing and configured to move distally about the needle and at least one actuation member. The actuation member is supported on the housing and has a proximal end secured to the housing, a distal end obstructing distal movement of the slide member and a centrally located fulcrum. A biasing member is positioned within the housing to advance the slide member distally along the needle. A cover member is operably connected to the slide member and is configured to enclose a distal end of the needle. The actuation member is configured such that actuation of the actuation member pivots the distal end of the actuation member to facilitate distal movement of the slide member. The distal end of the at least one actuation member includes an engagement member configured to obstruct distal movement of the slide member.

In one embodiment, the at least one actuation member includes a gripping portion for operable engagement by a user. The gripping portion may include a slip resistant surface such as knurls, ridges, grooves or the like.

The gripping portion is located proximally of the fulcrum and the engagement member is located distally of the fulcrum.

In one embodiment, a restraining member for restricting distal movement of the slide member is provided. The restraining member may include at least one tether. The at least one tether may include first and second spaced tethers.

In one embodiment, the at least one actuation member includes a pair of actuation members which are positioned on the housing at diametrically opposed positions.

In one embodiment, the proximal end of the housing is configured to releasably engage a syringe. The proximal end of the housing may include a luer-type connector.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a perspective view of one embodiment of the safety needle assembly of the present disclosure, in a first or armed position and operably connected to a syringe;

FIG. 2 is an enlarged perspective view of the safety needle assembly of FIG. 1, in a second or activated position;

FIG. 3 is a cross-sectional top view of the safety needle assembly of FIGS. 1 and 2, in the first or armed position along line 3-3 of FIG. 1;

FIG. 4 is an enlarged cross-sectional side view of the safety needle assembly of FIGS. 1-3, in the first or armed position along line 4-4 of FIG. 1;

FIG. 5 is a cross-sectional top view of the safety needle assembly of FIGS. 1 and 2, in the second or activated position;

FIG. 6 is an enlarged cross-sectional side view of the safety needle assembly of FIGS. 1-5, in the second or activated position;

FIG. 7 is an enlarged perspective view of an alternate embodiment of a safety needle assembly, in a first or armed position; and

FIG. 8 is an enlarged perspective view of another embodiment of a safety needle assembly, in a first or unarmed position.

### DETAILED DESCRIPTION

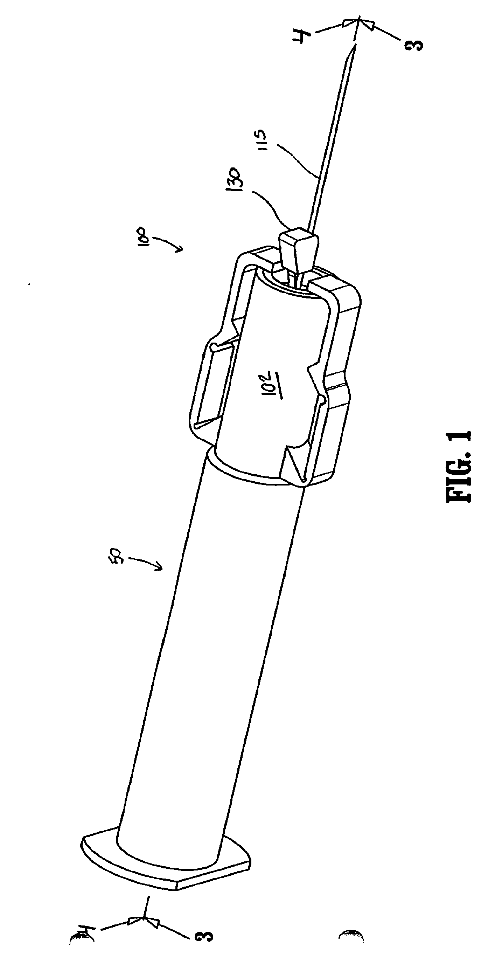

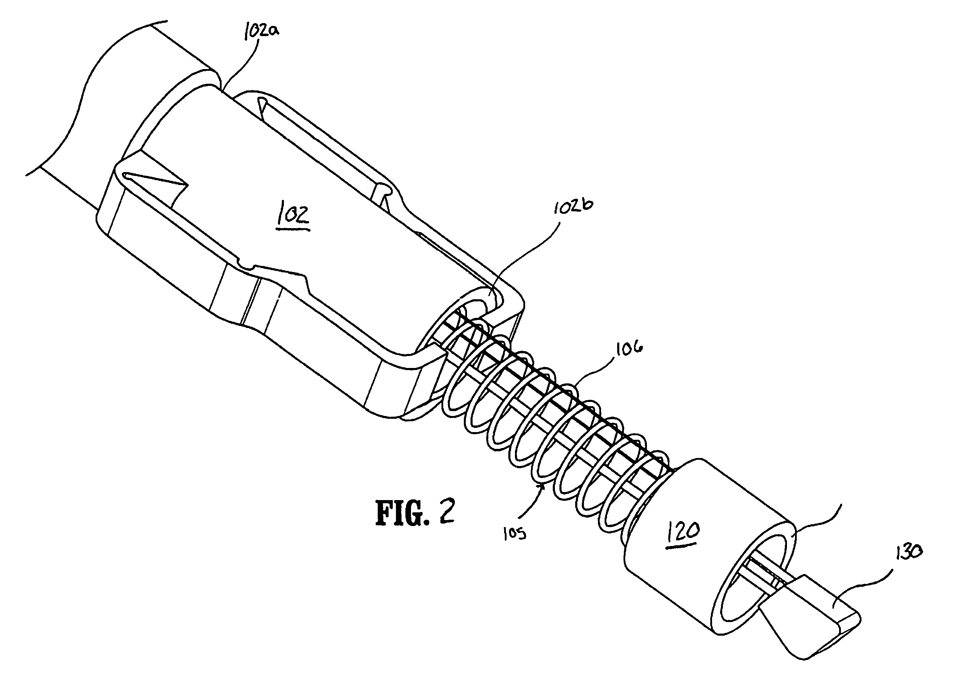

The present disclosure provides a safety needle assembly with a biased release mechanism. Referring initially to FIGS. 1 and 2, a safety needle assembly of the present disclosure is shown generally as safety needle assembly 100. Safety needle assembly 100 is configured for operable connection with a syringe 50. Although, as will be discussed in further detail below, safety needle assembly 100 is selectively removable from syringe 50, it is envisioned that safety needle assembly 100 may be integrally formed therewith.

Still referring to FIGS. 1 and 2, safety needle assembly 100 includes a housing 102, a needle 115 extending distally from housing 102, a slide member 120 configured to move distally along needle 115, and a cover member 130 operably connected to slide member 120 and configured to enclose a sharpened distal end 115b of needle 115.

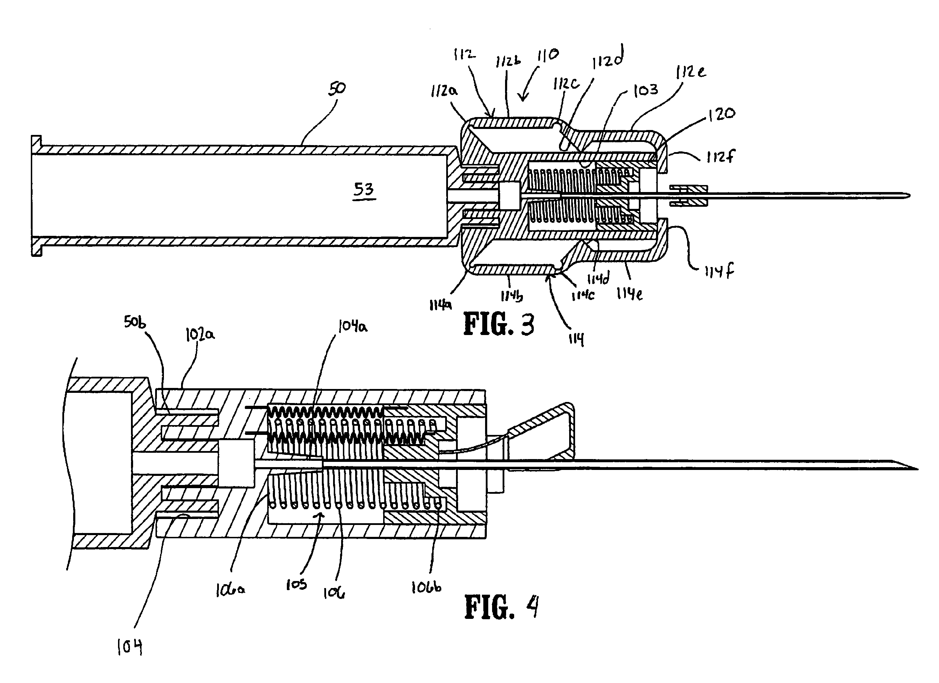

Housing 102 includes a substantially annular body having a substantially closed proximal end 102a and a substantially open distal end 102b. The annular body defines a cavity 103 (FIG. 3). Proximal end 102a of housing 102 is configured for selective engagement with a distal end 50b (FIG. 4) of syringe 50. As shown, distal end 50b of syringe 50 defines a luer-type connector which is configured to releasably engage a luer-type connector 104 (FIG. 4) which is formed on proximal end 102a of housing 102. Alternate configurations for connecting safety needle assembly 100 with syringe 50 are contemplated, e.g., friction fit, threading, or, as noted above, integrally forming safety needle assembly 100 with syringe 50. Needle 115 is mounted to a central hub portion 104a (FIG. 4) of housing 102 and extends distally from central hub portion 104a through distal end 102b of housing 102. Proximal end 102a of housing 102 is configured to fluidly connect needle 115 with a syringe cavity 53 (FIG. 3) of syringe 50. A first biasing member 105 is positioned within cavity 103 of housing 102 and extends distally about needle 115. In one embodiment, biasing member 105 may include a coil spring 106 (FIG. 4) having a proximal end 106a adjacent to proximal end 102a of housing 102 and a distal end 106b adjacent to slide member 120. Spring 106 is configured and positioned to be in compression about needle 115 when safety needle assembly 100 is in a first or armed position to urge safety needle assembly 100 to a second or activated position as will be discussed in further detail below.

With reference to FIG. 3, housing 102 includes a release mechanism 110 for selectively releasing slide member 120 from within cavity 103 of housing 102. Release mechanism 110 includes substantially identical first and second arms 112, 114. First and second actuation members or arms 112, 114 are mounted to proximal end 102a of housing 102 in a cantilevered fashion and extend distally along opposing sides thereof. First and second arms 112, 114 each include a first hinge portion 112a, 114a, a gripping portion 112b, 114b, a second hinge portion 112c, 114c, a fulcrum 112d, 114d, and a retaining portion 112e, 114e, respectively. Gripping portions 112b, 114b are bounded by first hinge portion 112a, 114a, respectively, on a proximal end thereof and by second hinge portion, 112c, 114c, respectively, on a distal end thereof. Gripping portions 112b, 114b are maintained in a spaced apart relationship from housing 102 by fulcrum 112d, 114d, respectively. Gripping portions 112b, 114b are configured for operable engagement by a user. Gripping portions 112b, 114b may include ridges, knurling, knobs or any other slip-resistant configuration. Retaining portions 112e, 114e extend distally from fulcrum 112d, 114d and include transversely extending engagement members 112f, 114f, respectively. Although two arms are illustrated, it is envisioned that one or more arms may be provided to actuate safety needle assembly 100 as will be discussed in further detail below.

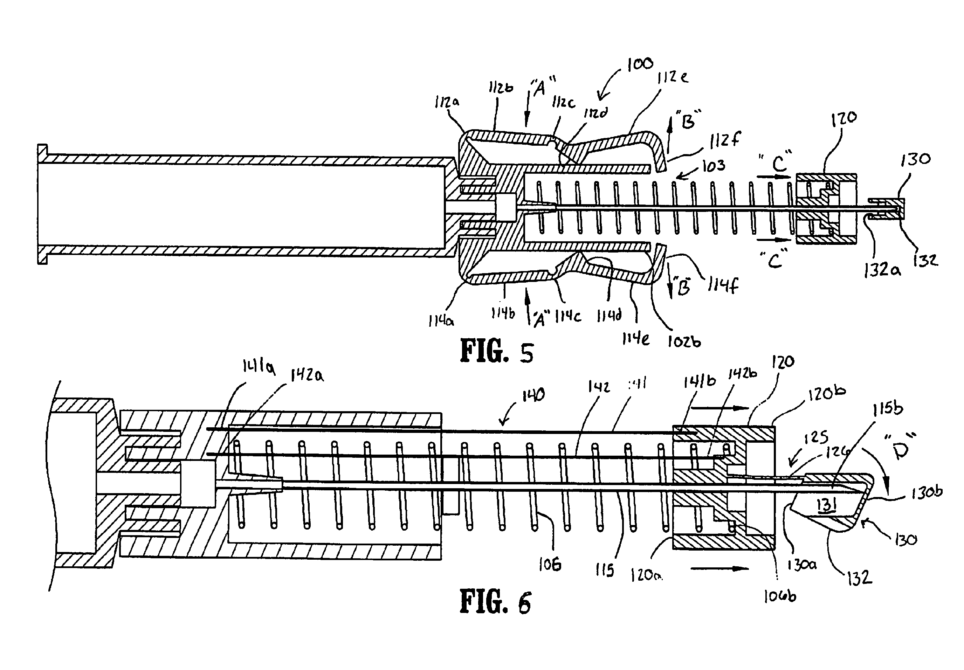

In a first or unbiased position, first and second arms 112, 114 are configured such that engagement members 112f, 114f extend across distal end 102b of housing 102. As will be discussed in further detail below, in this manner, engagement members 112f, 114f are positioned to obstruct distal movement of slide member 120, thereby retaining slide member 120 within cavity 103 of housing 102. Approximation of gripping portions 112b, 114b towards housing 102 as indicated by arrows "A" (FIG. 5) causes first and second arms 112, 114 to flex at first hinge portions 112a, 114a, respectively. As gripping portion 112b, 114b move towards housing 102, fulcrums 112d, 114d engage housing 102, thereby causing first and second arms 112, 114 to flex at second hinge portion 112c, 114c, respectively. Flexion at second hinge portions 112c, 114c permit first and second arms 112, 114 to pivot at fulcrums 112d, 114d, respectively. As first and second arms 112, 114 pivot at fulcrums 112d, 114d, respectively, retaining portions 112e, 114e, respectively, are pivoted away from housing 102. As retaining portions 112e, 114e pivot away from housing 102 as indicated by arrows "B" engagement members 112f, 114f are moved from across distal end 102b of housing 102 such that distal movement of slide member 120 is no longer obstructed.

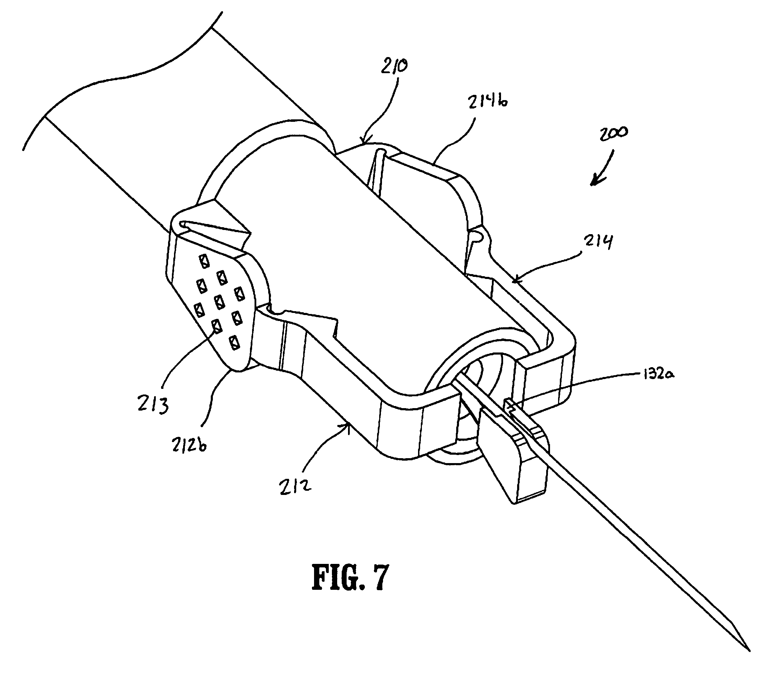

Turning now to FIGS. 6 and 7, slide member 120 has a substantially annular body configured to be received within cavity 103 of housing 102. Slide member 120 includes a proximal end 120a defining an opening 121 configured to slidably receive needle 115 and a substantially open distal end 120b. Proximal end 120a is configured to engage distal end 106b of spring 106. A biasing member 125 is mounted to and extends distally from slide member 120. As shown, biasing member 125 includes a leaf spring 126. Cover member 130 is mounted to a distal end of biasing member 125. As will be discussed in further detail below, biasing member 125 is configured to rotate cover member 130 over sharpened distal end 115a of needle 115, thereby protecting a user from an accidental needle stick.

Cover member 130 defines a substantially trapezoidal member having a substantially open proximal end 130a and a closed distal end 130b. Other configurations are envisioned. Cover member 130 further defines a cavity 131 for receiving distal end 115b of needle 115. Proximal end 120a of cover member 130 is operably engaged with biasing member 125. Alternatively, biasing member 125 may be integrally formed with cover member 130. Cover member 130 also defines a slot 132 along a length thereof. Slot 132 is configured to slidably receive needle 115 to allow cover member 130 to slide along the length of needle 115. As illustrated in FIG. 4, biasing member 125 urges cover member 130 into engagement with slot 132. Slot 132 includes an enlarged proximal end 132a. As will be discussed in further detail below, enlarged proximal end 132a of slot 132 permits distal end 115b of needle 115 to be received within cavity 131 of cover member 130 as cover member 130 approaches distal end 115b of needle 115.

Safety needle assembly 100 further includes a restraining member 140 for preventing slide member 120 from extending beyond distal end 115a of needle 115. In one embodiment, restraining member 140 may include at least one tether. As shown, restraining member 140 includes a first and second tether 141, 142. Proximal ends 141 a, 142a of first and second tether 141, 142 are secured to housing 102 at laterally spaced locations, while distal ends 141b, 142b of first and second tethers 141, 142 are secured to slide member 120 at laterally spaced locations. First and second tethers 141, 142 are secured to slide member 120 at laterally spaced locations to prevent rotation of slide member 120 about needle 115. First and second tethers 141, 142 are of sufficient length to permit slide member 120 to traverse along needle 115 to a position in which cover member 130 is positioned over distal end 115b of needle 115. In an alternative embodiment, it is envisioned that restraining means 140 may include a sleeve, pliable mesh covering or other suitable means for restricting the extension of spring 105.

With reference now to FIGS. 3-6, the operation of safety needle assembly 100 will be described in detail. Initially, safety needle assembly 100 is in a first or armed position. Slide member 120 is received about needle 115 and is selectively retained within cavity 103 of housing 102. Slide member 120 is biased distally by spring 105. Engagement portions 112f, 114f of first and second arms 112, 114, respectively, extend inwardly adjacent distal end 102b of housing 102, to retain slide member 120 in a retracted position located within cavity 103 of housing 102. In the armed position, gripping portions 112b, 114b of first and second arms 112, 114 are spaced generally parallel to housing 102. Needle 115 is received through slot 132 formed in cover member 130, and leaf spring 126 is positioned to bias cover member 130 towards needle 115.

With specific reference now to FIG. 5, safety needle assembly 100 is activated, when gripping portions 112b, 114b of first and second arms 112, 114 are approximated toward housing 102. This is typically accomplished by squeezing or pinching gripping portions 112b, 114b between a thumb and forefinger. In this manner, safety needle assembly may be operated with one hand. Additionally, since the force imparted by a user is directed toward the center of safety needle assembly 100, there is reduced likelihood of the user's fingers slipping off of release mechanism 110. This configuration also permits a user to activate release mechanism 110 while needle 115 remains within a patient without imparting axial movement of safety needle assembly 100 which may cause vascular damage and/or patient discomfort.

Approximation of gripping portions 112b, 114b towards housing 102 in a direction indicated by arrows "A", cause retaining portions 112e, 114e to pivot away from housing 102 about fulcrum 112d, 114d in the direction indicated by arrows "B", thereby retracting engagement members 112f, 114f from across distal end 102b of housing 102. Retraction of engagement members 112f, 114f permits the release of slide member 120 from within cavity 103 of housing 102. The extension of spring 105 causes slide member 120 to travel distally along needle 115 in the direction indicated by arrows "C". Tethers 141, 142 prevent the over extension of spring 105 to limit distal movement of slide member 120. Cover member 130 is also advanced distally with slide member 120. Needle 115 rides within slot 132 of cover member 130 until distal end 115b of needle 115 passes through proximal end 132a of slot 132. Once distal end 115b of needle 115 extends beyond slot 132 of cover member 130, lever 126 causes cover member 130 to rotate over distal end 115b of needle 115 (arrow "D"). In this manner, distal end 115b of needle 115 is received within cavity 131 of cover member 130. Cover member 130 may include a tab (not shown) for maintaining cover member 130 over distal end 115b of needle 115, thereby preventing accidental exposure thereof.

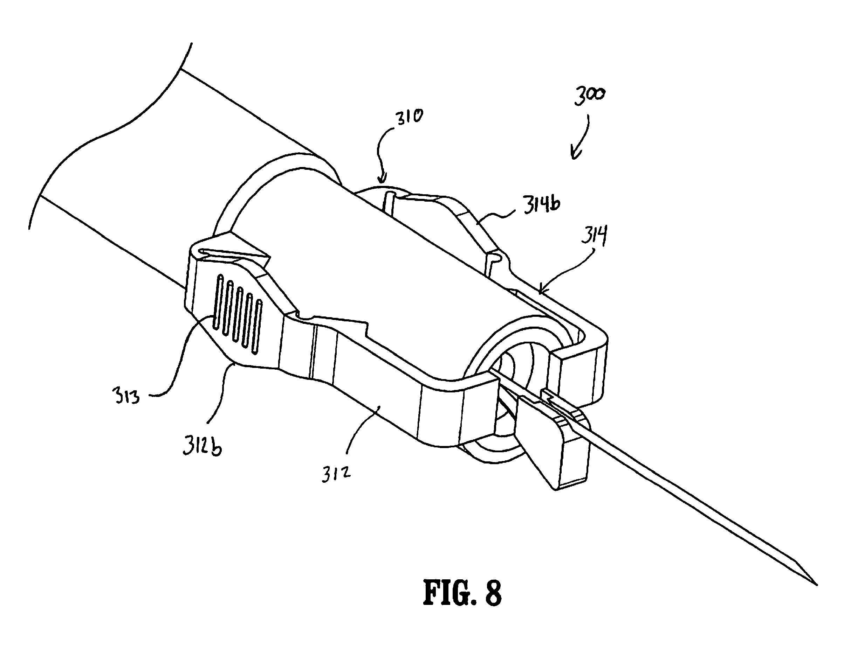

With reference now to FIGS. 7 and 8, alternate embodiments of the present disclosure are shown generally as safety needle assemblies 200, 300. Safety needle assemblies 200, 300 are substantially similar to safety needle assembly 100, and will only be described as relates to the differences therebetween. Safety needle assembly 200 includes a release mechanism 210. Release mechanism 210 includes first and second arms 212, 214, each having a gripping portion 212b, 214b, respectively. Gripping portions 212b, 214b include ridges 213 for facilitating engagement by a user. Safety needle assembly 300 also includes a release mechanism 310 having first and second arms 312, 314. Gripping portions 312b, 314b of first and second arms 312, 314 include teeth or knobs 313 for facilitating engagement by a user. It is envisioned that a variety of different textured gripping surfaces may be provided on the gripping portion of the first and second arms of the safety needle assembly to prevent slippage during actuation of the assembly.
Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure. For example, the biasing member for causing the extension of the slide member may include an elastic foam or pressurized cylinder.

## Claims

1. A safety needle assembly comprising:
a housing;
a needle extending distally from the housing;
a slide member positioned at least partially within the housing and configured to move distally about the needle;
at least one actuation member supported on the housing and having a proximal end secured to the housing, a distal end obstructing distal movement of the slide member and a centrally located fulcrum;
a biasing member positioned within the housing to advance the slide member distally along the needle; and
a cover member operably connected to the slide member, the cover member being configured to enclose a distal end of the needle, wherein actuation of the actuation member pivots the distal end of the actuation member to facilitate distal movement of the slide member.

2. The assembly according to claim 1, wherein the distal end of the at least one actuation member includes an engagement member configured to obstruct distal movement of the slide member.

3. The assembly according to any of the preceding claims, wherein the at least one actuation member includes a gripping portion for operable engagement by a user.

4. The assembly according to claim 3, wherein the gripping portion includes a slip resistant surface.

5. The assembly according to claim 4, wherein the slip resistant surface includes knurls.

6. The assembly according to claim 4, wherein the slip resistant surface includes ridges.

7. The assembly according to claim 4, wherein the slip resistant surface includes grooves.

8. The assembly according to any of claims 3-7, wherein the gripping portion is located proximal of the fulcrum and the engagement member is located distally of the fulcrum.

9. The assembly according to any of the preceding claims, further including a restraining member for restricting distal movement of the slide member.

10. The assembly according to claim 9, wherein the restraining member includes at least one tether.

11. The assembly according to claims 9 or 10, wherein the restraining member includes first and second spaced tethers.

12. The assembly according to any of the preceding claims, wherein the at least one actuation member includes a pair of actuation members.

13. The assembly according to claim 12, wherein the actuation members are positioned on the housing at diametrically opposed positions.

14. The assembly according to any of the preceding claims, wherein the proximal end of the housing is configured to releasably engage a syringe.

15. The assembly according to any of the preceding claims, wherein the proximal end of the housing includes a luer-type connector.
